# EUROPÄISCHE PATENTSCHRIFT

(11) **EP 3 585 328 B1**
(45) Veröffentlichungstag und Bekanntmachung des Hinweises auf die Patenterteilung: **10.04.2024**
(21) Anmeldenummer: 18710354.4
(22) Anmeldetag: 26.02.2018
(51) Int. Cl.: A61F 5/56

(54) **BEHANDLUNGSVORRICHTUNG ZUR INTRAORALEN ANWENDUNG UND BETRIEBSVERFAHREN EINER SOLCHEN BEHANDLUNGSVORRICHTUNG**
THERAPEUTIC DEVICE FOR INTRAORAL USE AND METHOD FOR OPERATING SUCH A THERAPEUTIC DEVICE
DISPOSITIF DE TRAITEMENT POUR L'UTILISATION INTRABUCCALE ET PROCÉDÉ DE FONCTIONNEMENT D'UN TEL DISPOSITIF DE TRAITEMENT

(30) Priorität: 24.02.2017 DE 102017103950
(43) Veröffentlichungstag der Anmeldung: 01.01.2020
(73) Patentinhaber: Berk, Bianca, 35037 Marburg (DE)
(72) Erfinder: KÖHLER, Jörg, 81245 München (DE); SAUER-SPERLING, Gotthard, 82319 Starnberg/Perchting (DE); BERK, Bianca, 82319 Starnberg/Perchting (DE)
(74) Vertreter: Oberdorfer, Jürgen
(86) Internationale Anmeldenummer: PCT/EP2018/054670
(87) Internationale Veröffentlichungsnummer: WO 2018/154110

(56) Entgegenhaltungen:
- WO-A1-2009/013371
- WO-A1-2013/117788
- WO-A1-2014/018105
- WO-A1-2014/018105
- WO-A2-00/44284
- DE-U1-202013 101 234
- DE-U1-202013 101 234
- ES-A2- 2 408 480
- US-A1- 2010 204 747
- US-A1- 2010 204 747
- US-A1- 2015 045 705
- US-A1- 2015 045 705

## Beschreibung

Die Erfindung betrifft eine Behandlungsvorrichtung zur intraoralen Anwendung nach dem Oberbegriff des Anspruchs.

Aus der DE 10 2010 005 359 A1 ist eine Knirscherschiene sowie ein Verfahren zur Herstellen dieser Knirscherschienen bekannt. Derartige Knirscherschienen weisen eine im Querschnitt U-förmige, sich durchgängig entlang einem Kieferbogen erstreckende Schienenleiste auf, welche auf Zähne eines Kieferbogens aufsteckbar ist. Alle freien Zahnenden entlang des Verlaufs der Schiene werden durch die Schiene abgedeckt Weiterhin werden Knirscherschienen angegeben, welche aus singulären Befestigungseinrichtungen zum Aufstecken auf Backenzähne gebildet sind. Weiterhin ist z. B. eine Auslösecharakteristik und eine Stimulationsstärke fest vorgegeben.

Aus der DE 10 2004 009 883 ist eine Vorrichtung zur Behandlung des Kiefergelenks bekannt. Eine derartige Vorrichtung weist kissenartige, mit Flüssigkeit gefüllte Polster auf, welche über einer Überströmleitung miteinander verbunden sind und bei unterschiedlicher Druckbeaufschlagung der Kissen für einen Kraftausgleich auf den Kiefer sorgen soll. Eine derartige Vorrichtung zur Behandlung des Kiefergelenkes ist nicht geeignet, um beispielsweise das Zungenpressen oder das Zähneknirschen, sofern es im pathologischen Ausmaß vorliegt, zu vermindern.

Aus der US 4,976,618 ist eine intraorale Sensoreinrichtung bekannt, welche Druckschläuche aufweist, die zwischen Zähnen des Ober- und Unterkiefers angeordnet sind und eine Signalleitung zur Weitergabe eines solchen Drucksignales an ein externes Gerät besitzt. Das externe Gerät ist außerhalb des Mundes angeordnet. Weiterhin ist das externe Gerät mit einem Lautsprecher verbunden, der beispielsweise als Ohrhörer ausgebildet ist. Eine derartige Sensorvorrichtung ist umständlich zu tragen und findet bei entsprechenden Anwendern oft nur geringe Akzeptanz.

Aus der US 2015/0045705 A1, die den nächstliegenden Stand der Technik darstellt, ist eine gattungsgemäße Behandlungsvorrichtung bekannt.

Aus der WO 2014/018105 A1 ist eine Mundorthese in der Form einer Aufbissschiene bekannt, welche abstandshaltende Platten zwischen einem Oberkiefer und einem Unterkiefer des Verwenders vorsieht. Diese Oralorthese ist passiv und verfügt über keine Biofeedbackfunktionen.

Aufgabe der Erfindung ist es, eine Behandlungsvorrichtung zur intraoralen Anwendung anzugeben, welche einen hohen Tragekomfort sowie einen hohen Behandlungserfolg, insbesondere zur Verminderung des Zungenpressens oder des Zähneknirschens oder des Schnarchens bietet und insbesondere bequem und als wenig störend empfunden auch im Schlaf getragen werden kann.

Eine weitere Aufgabe der Erfindung ist es, eine solche Behandlungsvorrichtung zur intraoralen Anwendung besonders kostengünstig und einfach herstellbar zu machen, insbesondere eine Behandlungsvorrichtung anzugeben, welche für eine Vielzahl von unterschiedlichen Anwendern passt oder leicht an deren Physionomie des Mundraumes anpassbar ist, so dass zumindest hinsichtlich der Geometrie und der Raumform der Behandlungsvorrichtung für eine Vielzahl von Anwenders eine Einheitsgröße und eine Einheitsraumform angegeben werden kann.

Diese Aufgaben werden mit einer Behandlungsvorrichtung zur intraoralen Anwendung mit den Merkmalen des Anspruchs 1 gelöst. Vorteilhafte Ausführungsformen sind in den Unteransprüchen angegeben.

Erfindungsgemäß weist eine Behandlungsvorrichtung zur Behandlung zur intraoralen Anwendung, insbesondere von Zähneknirschen und/oder Zungenpressen und/oder Schnarchen zumindest auf:
- zumindest zwei Befestigungseinrichtungen zum Festlegen der Behandlungsvorrichtung an mindestens jeweils einem Zahn eines Oberkiefers und/oder Unterkiefers eines Anwenders;
- mindestens eine Sensoreinrichtung ausgebildet und eingerichtet zur Detektion von Druckbelastungsereignissen und/oder Schallereignissen und/oder Schwingungsereignissen im Mundraum des Anwenders;
- mindestens eine Stimulationseinrichtung zur Stimulation des Anwenders, wenn ein Druckbelastungsereignis und/oder ein Schallereignis und/oder ein Schwingungsereignis auftritt sowie
- eine Steuerungs- und/oder Regelungseinheit zum gesteuerten und/oder geregelten Betreiben der Stimulationseinrichtung in Abhängigkeit vom auftretenden Druckbelastungsereignis und/oder Schallereignis und/oder Schwingungsereignis sowie
- eine Energieversorgung.

Sie ist weiterhin dadurch gekennzeichnet, dass die Befestigungseinrichtungen mit mindestens einem biegsamen Verbindungssteg miteinander verbunden sind, der dazu eingerichtet und ausgebildet ist, im bestimmungsgemäßen Gebrauch der Behandlungsvorrichtung an Zahnaußenseiten und/oder Zahninnenseiten unter Freilassung von freien Zahnenden der Zähne entlang denen sich der Verbindungssteg erstreckt, anzuliegen, wobei insbesondere zumindest alle vorgenannten Bestandteile der Behandlungsvorrichtung im bestimmungsgemäßen Gebrauch der Behandlungsvorrichtung intraoral angeordnet sind. Weiterhin ist die mindestens eine Sensoreinrichtung und/oder eine Signalleitung im Verbindungssteg integriert oder jeder Sensoreinrichtung ist eine Steuerungs-/Regeleinheit eine Stimulationseinrichtung und eine Energieversorgung zugeordnet, so dass jede Befestigungseinrichtung eine autark funktionierende Anordnung aus Sensoreinrichtung, Steuerungs-/Regeleinheit, Stimulationseinrichtung und Energieversorgung besitzt.

Mit einer solchen Behandlungsvorrichtung zur intraoralen Anwendung ist sichergestellt, dass Zähne des Oberkiefers oder des Unterkiefers, je nachdem an welchem Kiefer die Behandlungsvorrichtung im bestimmungsgemäßen Gebrauch angeordnet ist, an ihren freien Enden frei bleiben. Dies wird gegenüber dem Stand der Technik vom Anwender als komfortabel empfunden. Der biegsame Verbindungssteg sorgt außerdem insbesondere dafür, dass die Haltevorrichtungen in ihrem seitlichen Abstand zueinander in weiten Grenzen und an die physiologischen Randbedingungen des Mundraumes des Anwenders anpassbar einstellbar sind, so dass die Behandlungsvorrichtung einfach z. B. an unterschiedliche Kiefergeometrien unterschiedlicher Anwender anpassbar ist.

Des Weiteren wird insbesondere durch das bevorzugt vorgespannte Anliegen des Verbindungssteges entweder an Zahnaußenseiten (dies wiederum bevorzugt, wenn die Behandlungsvorrichtung am Oberkiefer getragen wird) oder an Zahninnenseiten (dies bevorzugt, wenn die Behandlungsvorrichtung auf dem Unterkiefer getragen wird) ein straffer Sitz der Behandlungsvorrichtung im Mund des Anwenders sichergestellt. Zudem ist eine Ausgestaltung möglich, bei der unabhängig vom Trageort der Behandlungsvorrichtung ein innerer und/oder äußerer Verbindungssteg vorgesehen ist.

Ein Abrutschen des biegsamen Verbindungssteges in schmerzempfindliche Bereiche, beispielsweise in Bereiche des Zahnfleisches ist zuverlässig verhindert.

Weiterhin ist durch das Anliegen des Verbindungssteges an freien Zahnaußenseiten ein beabsichtigtes Verrutschen der Behandlungsvorrichtung im Mund des Anwenders nach hinten zuverlässig verhindert. Bei einem Anliegen des Verbindungssteges an Zahninnenseiten eines Kiefers ist ein unbeabsichtigtes Verrutschen der Behandlungsvorrichtung nach vorne zuverlässig unterbunden. Es kann auch ein doppelter Verbindungssteg verwendet werden, der an der Zahnaußenseite und Zahninnenseite läuft.

Somit ist für eine sichere Positionierung der Behandlungsvorrichtung im Mundraum des Anwenders gesorgt, obwohl es sich in einer besonderen Ausführungsform der Erfindung um eine "one size fits all"- Behandlungsvorrichtung handelt, d. h. um zumindest um eine Einheitsgröße, welche derart ausgestaltet ist, dass sie auf eine möglichst große Vielzahl von Anwenderkiefern oder Anwendermundräume passt. Die erfindungsgemäße Behandlungsvorrichtung ist somit, ohne ihre Raumform bei der Herstellung individuell an Anwenderkiefer anpassen zu müssen, tragbar und funktionsfähig, so dass eine kostengünstige Großserienherstellung der Behandlungsvorrichtung ermöglicht ist.

In einer weiteren Ausführungsform der Behandlungsvorrichtung sind die Befestigungseinrichtungen kappenartig oder im Querschnitt U-förmig nach Art einer U-Schiene ausgebildet und übergreifen mindestens einen Zahn, bevorzugt einen Backenzahn, wenn die Behandlungsvorrichtung vom Anwender getragen wird. In diesem Zusammenhang sei angemerkt, dass unter dem Begriff "bestimmungsgemäßer Gebrauch" erfindungsgemäß zu verstehen ist, dass die Behandlungsvorrichtung bzw. deren Bestandteile derart eingerichtet und ausgebildet sind, dass sie beim ordnungsgemä-βen und beabsichtigten Tragen der Behandlungsvorrichtung durch einen Anwender, beispielsweise durch Aufstecken der Behandlungsvorrichtung auf Zähne des Oberkiefers oder auf Zähne des Unterkiefers ihre zugedachten Aufgaben lösen und Eigenschaften entfalten können. Als bestimmungsgemäßer Gebrauch ist somit der Einsatz der Behandlungsvorrichtung im Mundraum eines Anwenders im aufgesteckten Zustand zu verstehen.

Bei einer weiteren bevorzugten Ausführungsform der Behandlungsvorrichtung besitzt die Behandlungsvorrichtung mindestens einen Wandungsabschnitt, der im bestimmungsgemäßen Gebrauch zwischen Zähnen des Oberkiefers und des Unterkiefers des Anwenders angeordnet ist und die mindestens eine Sensoreinrichtung in diesem Wandungsabschnitt integriert ist.

Unter "integriert" ist erfindungsgemäß zu verstehen, dass die Sensoreinrichtung entweder in den Wandungsabschnitt, der zwischen den Zähnen des Oberkiefers und des Unterkiefers angeordnet ist, eingebettet ist oder diesen bildet. Ebenso ist denkbar, dass die Sensoreinrichtung auf diesem Wandungsabschnitt oder unter diesem Wandungsabschnitt liegend befestigt ist.

Durch eine derartige Anordnung gelingt eine zuverlässige Detektion beispielsweise von Druckereignissen im Mundraum des Anwenders, wie sie beispielsweise durch das Zähneknirschen entstehen.

In einer weiteren Ausführungsform der Erfindung ist die mindestens eine Sensoreinrichtung im Verbindungssteg integriert. Außerdem kann bei der Erfindung, insbesondere wenn der Verbindungssteg entlang von Innenseiten von Zähnen, beispielsweise des Unterkiefers verläuft, kann in einfacher Art und Weise ein Zungenpressen, welches üblicherweise durch ungewolltes Andrücken der Zunge an vordere Zähne stattfindet, detektiert werden.

Auch kann alternativ oder zusätzlich eine Signalleitung im Verbindungssteg integriert sein, um beispielsweise eine erste Drucksensoreinrichtung in einem der Wandungsabschnitte mit einer zweiten Drucksensoreinrichtung in einem anderen der Wandungsabschnitte zu verbinden und/oder weitergehend mit der Steuer- und/oder Regeleinrichtung zu verbinden.

Weiter alternativ ist die Steuerungseinheit und/oder die Regelungseinheit und/oder die Stimulationseinrichtung und/oder die Energieversorgung innenseitig in eine an zumindest einer der Befestigungseinrichtungen angeformte Anformung integriert oder eingekapselt, insbesondere eingegossen.

Als Stimulationseinrichtungen kommen alle Arten von geeigneten Stimulatoren in Frage. Insbesondere seien hier Vibratoren, z. B. Umwuchtmotoren und/oder Piezoschwinger erwähnt. Des Weiteren können als Stimulatoren auch Elektroden vorgesehen sein, welche eine elektrische Stimulation bewirken. Außerdem kann als Stimulator auch eine Schallquelle, beispielsweise ein Lautsprecher oder dergleichen verwendet werden.

In einer weiteren Ausführungsform der Behandlungsvorrichtung ist die Anformung korrespondierend zu einem Gaumengewölbe ausgeformt oder an ein Gaumengewölbe zumindest teilweise anpassbar ausgebildet oder ist einem standardisierten Gaumengewölbe, welches einem Mittelgewölbe einer möglichst großen Anzahl von unterschiedlichen Anwendern entspricht, angepasst.

Diese Maßnahme erhöht den Tragekomfort und die Anwendbarkeit der erfindungsgemäßen Behandlungsvorrichtung für eine Vielzahl von Anwendern bei gleicher oder ähnlicher Gesamtraumform der Behandlungsvorrichtung.

In einer weiteren Ausführungsform der Behandlungsvorrichtung ist der Verbindungssteg bezüglich einer Krümmung entlang der Zahninnenseite oder der Zahnaußenseite, d. h. um eine Hochachse (H) biegeweicher ausgestaltet ist, als um eine Querachse (Q), die beispielsweise senkrecht zum Verlauf des Verbindungsstegs, insbesondere senkrecht auf eine Breitseite des Verbindungssteges gerichtet ist. Hierdurch bei einem etwas lockereren Sitz der Behandlungsvorrichtung im Mundraum eines Anwenders ein unbeabsichtigtes Durchhängen des Verbindungssteges und somit ein ungewolltes Berühren oder Scheuern des Zahnfleisches durch den Verbindungssteg gut verhindert.

In einer besonderen Ausführungsform der Behandlungsvorrichtung ist der Verbindungssteg aus einem gummiartig dehn- und/oder stauchbaren Material ausgebildet und/oder weist einen Randbeschnitt auf, welcher ein Ziehharmonika-artiges Dehnen und/oder Stauchen erlaubt. Mit dieser Ausgestaltung ist es in besonders einfacher Art und Weise möglich, die Behandlungsvorrichtung in einem mechanisch etwas vorgespannten Zustand in den Mund des Anwenders einzusetzen. Insbesondere wenn keine Signalleitung durch den Steg verläuft, kann dies eine besonders bevorzugte Ausführungsform sein. Wenn eine Signalleitung durch den Verbindungssteg verläuft, so muss diese selbstverständlich derart ausgestaltet sein, dass sie entsprechende Dehnungen und Stauchungen mitvollziehen kann, ohne Schaden zu nehmen.

In einer weiteren Ausführungsform der Behandlungsvorrichtung ist der Verbindungssteg zugelastisch ausgebildet, wenn der Steg im bestimmungsgemäßen Gebrauch zur Anlage an der Zahnaußenseite vorgesehen ist und/oder druckelastisch, stauchbar ausgebildet ist, wenn der Verbindungssteg im bestimmungsgemä-βen Gebrauch zur Anlage an der Zahninnenseite ausgebildet ist.

Diese Maßnahmen tragen zu einem verbesserten Tragekomfort und auch zu einer verbesserten Sensierung oder Detektierung von Druck-und/oder Schall und/oder Schwingungsereignissen im Mundraum bei, da für einen definierten und festen Sitz der Behandlungsvorrichtung im Mundraum gesorgt ist.

Im Folgenden wird die Erfindung anhand der Zeichnung beispielhaft näher erläutert. Es zeigen:
- Figur 1:: stark schematisiert eine erste Ausführungsform der Behandlungsvorrichtung zur intraoralen Anwendung in perspektivischer Ansicht im aufgesteckten Zustand auf einen Kiefer des Anwenders, mithin im Zustand des bestimmungsgemäßen Gebrauchs;
- Figur 2:: die Behandlungsvorrichtung gemäß Figur 1 in einer Draufsicht;
- Figur 3:: eine Draufsicht auf eine zweite Ausführungsform der erfindungsgemäßen Behandlungsvorrichtung zur intraoralen Anwendung;
- Figur 4:: eine stark schematisierte perspektivische Ansicht auf eine dritte Ausführungsform der erfindungsgemäßen Behandlungsvorrichtung zur intraoralen Anwendung;
- Figur 5:: eine perspektivische, stark schematisierte Ansicht auf eine vierte Ausführungsform der erfindungsgemäßen Behandlungsvorrichtung zur intraoralen Anwendung.

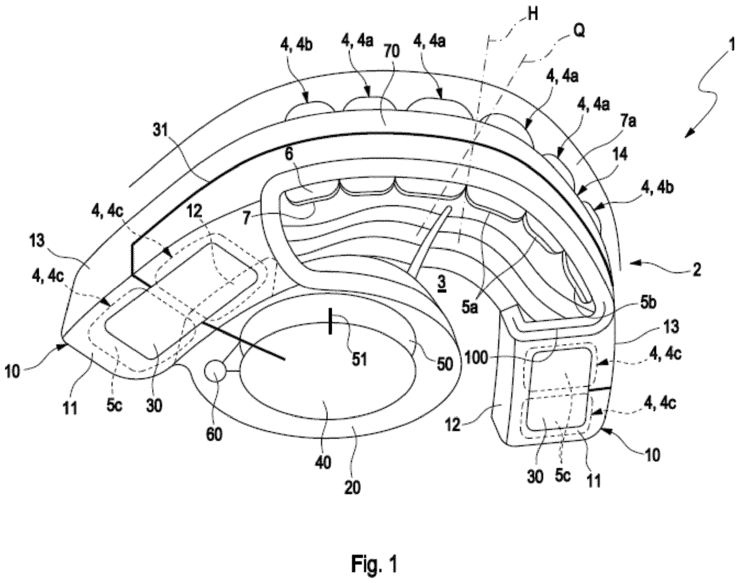

Eine erfindungsgemäße Behandlungsvorrichtung 1 zur intraoralen Anwendung ist in den Figuren 1 in einer ersten Ausführungsform schematisch dargestellt.

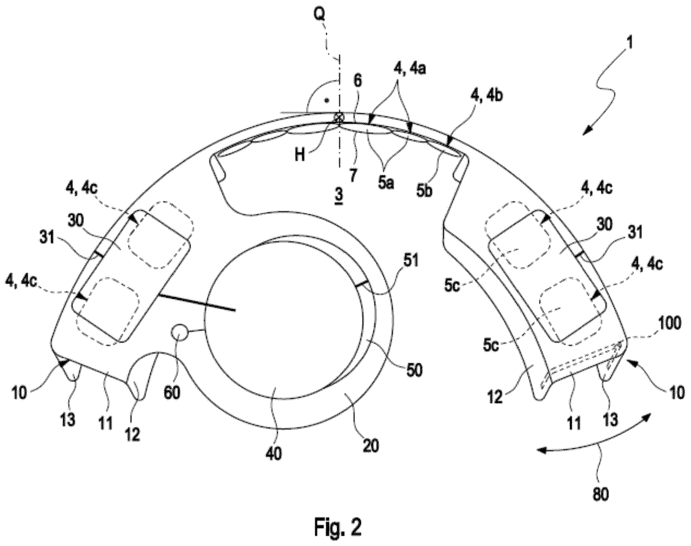

Figur 1 zeigt eine perspektivische Darstellung der Behandlungsvorrichtung 1 eingesetzt bzw. aufgesteckt auf einen Kiefer 2 eines Anwenders. Das Ausführungsbeispiel gemäß Figuren 1 und 2 zeigt dabei die Behandlungsvorrichtung 1 aufgesteckt auf den Oberkiefer des Anwenders, was durch die schematische Darstellung eines Gaumens 3 verdeutlicht ist. Der Kiefer 2, hier der Oberkiefer, weist Zähne 4 auf. Insbesondere sind schematisch dargestellt Frontzähne 4a, Eckzähne 4b und Backenzähne 4c. Die Zähne 4 weisen freie Enden 5 auf, wobei freie Enden der Frontzähne 5a, freie Enden der Eckzähne 5b und freie Enden von Backenzähnen 5c verdeutlicht sind. Die Zähne 4 weisen Außenseiten 6 und Innenseiten 7 auf, welche dem Gaumen 3 bzw. einem Gaumengewölbe des Gaumens 3 zugewandt sind.

Ebenfalls schematisch dargestellt ist ein Zahnfleisch 7a.

Die Behandlungsvorrichtung 1 zur intraoralen Anwendung besitzt zwei Befestigungseinrichtungen 10, welche im Querschnitt U-förmig nach Art von U-Schienen ausgebildet sind und weiterhin eingerichtet und ausgebildet sind, um auf zumindest einen Backenzahn 4c, bevorzugt mehrere Backenzähne 4c diese umgreifend aufgesteckt zu werden. Die Befestigungseinrichtungen weisen dabei zumindest einen Wandungsabschnitt 11 auf, welcher im bestimmungsgemäßen Gebrauch der Behandlungsvorrichtung 1 die freien Enden 5c der Backenzähne 4c abdeckt, so dass dieser Wandungsabschnitt 11 in bestimmungsgemäßem Gebrauch der Behandlungsvorrichtung beim Zubeißen des Anwenders einer Quetschung zwischen den Backenzähnen des Oberkiefers und entsprechenden Backenzähnen des Unterkiefers ausgesetzt ist.

Des Weiteren weisen die Befestigungseinrichtungen 10 innere Wandungsabschnitte 12 auf, welche an den Backenzähnen 4c innenseitig, d. h. gaumenseitig bevorzugt klemmend anliegen.

Des Weiteren besitzen die Befestigungseinrichtungen äußere Wandungsabschnitte 13, welche auf Außenseiten der Backenzähne 4c anliegen, so dass die Wandungsabschnitte 11, 12, 13 nach Art einer Klammer mindestens einen Backenzahn übergreifen und so als Befestigungseinrichtung 10 für die Behandlungsvorrichtung 1 wirken.

Die Befestigungseinrichtungen 10 sitzen jeweils bevorzugt gegenüberliegend auf Backenzähnen 4c eines Kieferhalbbogens. Die Befestigungseinrichtungen sind mit mindestens einem biegsamen Verbindungssteg 14 miteinander verbunden, wobei sich der Verbindungssteg 14 im bestimmungsgemäßen Gebrauch entlang der Außenseiten 6 (gezeigt in Figur 1) der Frontzähne 5a und gegebenenfalls der Eckzähne 5b erstreckt, und dort anliegt, insbesondere vorgespannt anliegt. Der Verbindungssteg 14 lässt dabei die freien Enden der Frontzähne 5a, der Eckzähne 5b und der gegebenenfalls nicht vom Wandungsabschnitt 11 abgedeckten Backenzähne 5c frei. Der Verbindungssteg 14 ist darüber hinaus derart angeordnet und ausgebildet, dass möglichst keine Berührung des Zahnfleisches 7a im bestimmungsgemäßen Gebrauch erfolgt.

Weiterhin weist die Behandlungsvorrichtung 1 eine Anformung 20 auf, welche beispielsweise einstückig mit einer der Befestigungseinrichtungen 10 verbunden ist bzw. an diese angeformt ist. Es ist bevorzugt, die Anformung 20 lediglich an einer der Befestigungseinrichtungen 10 anzuformen, so dass die andere Befestigungseinrichtung 10 über den biegsamen Verbindungssteg 14 relativ leicht bezüglich, der Befestigungseinrichtung 10 mit der Anformung 20 beweglich ist, so dass eine gute Anpassbarkeit an verschiedene Kiefergeometrien unterschiedlicher Anwender erfolgen kann. In anderen Worten kann der seitliche Abstand zwischen den Befestigungseinrichtungen 10 durch die biegsame, insbesondere die federnd biegsame Ausgestaltung des Verbindungssteges in weiten Grenzen an die physiologischen Gegebenheiten im Mundraum eines Anwenders angepasst werden.

In dem Wandungsabschnitt 11, der im Falle eines Zubeißens des Anwenders zwischen den Backenzähnen 5c des Oberkiefers und Backenzähnen eines Unterkiefers angeordnet ist und somit einer Quetschung ausgesetzt ist, ist eine Sensoreinrichtung 30 eingebettet oder angeordnet. Die Sensoreinrichtung 30 kann beispielsweise ein Drucksensor, z. B. ein kraftsensierender Wiederstand sein. Er kann allerdings auch ohne weiteres als aktiver Drucksensor ausgebildet sein, z. B. als Piezosensor, welche bei Verformung unter Krafteinwirkung eine elektrische Spannung liefert. In einem einfachsten Fall kann die Sensoreinrichtung 30 auch als Druckschalter ausgebildet sein, welcher lediglich einen eingeschalteten und einen ausgeschalteten Zustand wiederspiegeln kann. Bevorzugt sind ist die Sensoreinrichtungen 30 allerdings derart ausgebildet, dass ein Sensorsignal gebildet wird, welches sich in Abhängigkeit der Druckbeaufschlagung ändert. Eine solche Änderung kann proportional oder einer anderen Funktion folgend, z. B. logarithmisch erfolgen.

Grundsätzlich reicht es aus, lediglich in einem Wandungsabschnitt 11 einer der beiden Befestigungseinrichtungen 10 eine solche Drucksensoreinrichtung 30 vorzusehen. Bevorzugt ist es allerdings, in beiden Befestigungseinrichtungen 10 derartige Sensoreinrichtungen 30 vorzusehen, um auch bei einer ungleichmäßigen Belastung der Kieferbögen, beispielsweise durch einen schiefen Biss eine zuverlässige Sensierung von Druckereignissen oder dergleichen zur Verfügung zu stellen.

Wenn z. B. in jeder Befestigungseinrichtung 10 eine solche Sensoreinrichtung 30 vorgesehen ist, so empfiehlt es sich entsprechende Sensorsignalleitungen 31 zur Anbindung derjenigen Sensoreinrichtung 30, die nicht in der Nähe der Anformung 20 angeordnet ist, vorzusehen. Eine derartige Signalleitung 31 kann zweckmäßiger Weise entlang des Verbindungssteges 14 vorgesehen werden, insbesondere in diesen eingebettet sein.

Die Sensoreinrichtungen 30 stehen mit einer Steuerungs-/Regeleinheit 40 in Verbindung, welche dazu eingerichtet und ausgebildet ist, Sensorsignale der zumindest einen Sensoreinrichtung 30 zu empfangen und auszuwerten.

Des Weiteren weist die Behandlungsvorrichtung zur intraoralen Anwendung eine Stimulationseinrichtung 50 auf, welche über eine schematisiert angedeutete Verbindung 51 von der Steuerungs-/Regeleinheit 40 angesteuert werden kann. Die Stimulationseinrichtung 50 kann beispielsweise eine mechanische und/oder elektrische Stimulationseinrichtung sein. Ferner kann die Stimulationseinrichtung auch eine thermisch wirkende oder als Schallquelle wirkende Stimulationseinrichtung ausgebildet sein. Als mechanisch ausgebildete Stimulationseinrichtung kommen beispielsweise Umwuchtmotoren oder Piezo-Schwinger in Betracht. Als elektrisch wirkende Stimulationseinrichtungen kommen beispielsweise Reizelektroden in Betracht, welche im bestimmungsgemäßen Gebrauch der Behandlungsvorrichtung 1 beispielsweise Kontakt mit dem Zahnfleisch oder dem Gaumen oder dem Wangenfleisch des Anwenders haben, um dort gegebenenfalls elektrische Reize auszulösen.

Eine thermisch wirkende Stimulationseinrichtung 50 kann beispielsweise ein Heizelement oder Kühlelement, z. B. ein Peltierelement sein, welches an bestimmten Stellen, beispielsweise auch am Gaumen oder am Zahnfleisch des Anwenders einen Wärme- und/oder Kältereiz erzeugt.

Selbstverständlich kann die Stimulationseinrichtung 50 auch als Schallquelle ausgebildet sein, um eine akustische Stimulation des Anwenders, beispielsweise durch einen Piepston oder Brummton, hervorzurufen.

Des Weiteren weist die Behandlungsvorrichtung 1 eine Energieversorgung 60 auf, welche zur Versorgung der Steuerungs-/Regeleinheit der Stimulationseinrichtung(en), der Sensoreinrichtungen, soweit erforderlich verwendet wird.

Die vorbeschriebene Ausführungsform der Behandlungsvorrichtung 1 eignet sich beispielsweise, um ein unbeabsichtigtes Zähneknirschen im Schlaf eines Anwenders zu detektieren und mit geeigneter Stimulation zu behandeln, insbesondere dahingehend, dass eine Verminderung, gegebenenfalls sogar eine Verhinderung des Zähneknirschens aufgrund eines Biofeedbacks und eines hieraus resultierenden Lernprozesses des Anwenders, der auch im Unterbewusstsein stattfinden kann, erfolgt.

Sofern die Behandlungsvorrichtung 1 zur Bekämpfung von ungewolltem Schnarchen oder dergleichen eingesetzt werden soll, können die Sensoreinrichtungen auch als Mikrofone zur Aufnahme von Schallereignissen oder anderweitigen Schwingungsereignissen im Mundraum ausgebildet sein.

Ebenso kann es zweckmäßig sein, entsprechende Sensoreinrichtungen 30, beispielsweise Drucksensoren oder dergleichen an Stellen vorzusehen, an denen ein sogenanntes Zungenpressen, also ein verkrampfendes Andrücken der Zunge im Schlaf an eine bestimmte Stelle im Mundraum, z. B. die Zahninnenseiten 7 stattfindet. Ein derartiges Zungenpressen kann so weit gehen, dass sich mit der Zeit sogar die Lage der Zähne 4 im Kiefer verändert. Das Zungenpressen führt in vielen Fällen oftmals zu Überanstrengungsreaktionen der entsprechenden Muskulatur und gegebenenfalls Kopfschmerzen, Nackenschmerzen oder anderen Beschwerden des Anwenders.

Des Weiteren ist vorgesehen, dass zumindest die Befestigungseinrichtungen 10, der Verbindungssteg 14, die Anformung 20 enthaltend die Steuerungs-Regeleinheit und die Stimulationseinrichtung sowie die Sensoreinrichtungen 30 und gegebenenfalls vorhandene Signalleitungen 31 und die Energieversorgung 60 im bestimmungsgemäßen Gebrauch allesamt intraoral angeordnet sind, was verhindert, dass z. B. Kabel und/oder Antennen und/oder dergleichen andere Bauteile aus dem Mund herausgeführt werden müssen. Dies wird oftmals von Anwendern als sehr unbequem und auch unästhetisch empfunden. Eine vollständige intraorale Anordnung der Behandlungsvorrichtung 1 wirkt diesen als nachteilig empfundenen Eigenschaften von Behandlungsvorrichtungen aus dem Stand der Technik entgegen.

Der Verbindungssteg 14 kann grundsätzlich eine beliebe Querschnittsraumform besitzen. Besonders bevorzugt ist es allerdings, dass ein Querschnittsverlauf des Verbindungssteges 14 gewählt wird, welcher bezüglich einer Biegung um eine Hochachse H biegeweicher ausgestaltet ist als um eine Querachse Q. Die Hochachse H ist dabei so zu verstehen, dass sie in etwa parallel zu den Längserstreckungen der Zähne vom Zahnfleisch bis zu deren freien Enden 5a ausgerichtet ist. Eine Querachse Q ist so zu verstehen, dass sie beispielsweise senkrecht auf einer Zahnaußenseite 16 und somit senkrecht auf einer Außenseite 70 des Verbindungsschenkels oder einer entsprechenden Innenseite steht. Wesentlich ist, dass die unterschiedliche Biegbarkeit in einer Richtung um die Hochachse H (biegeweichere Ausgestaltung) im Gegensatz zu einer Biegung um eine Achse Q dazu führt, dass der Verbindungssteg 7 in einer Hochrichtung stabil ausgebildet ist und zuverlässig auf den Zahnaußenseiten 6 angelegt bleibt. Hierdurch ist zum einen ein guter Sitz der Behandlungsvorrichtung 1 im Mundraum gewährleistet. Andererseits ist auch zuverlässig verhindert, dass der Verbindungssteg 14 beispielsweise am Zahnfleisch 7a scheuern und somit für eine Reizung des Zahnfleisches 7a sorgen könnte. Es ist zu erwarten, dass dies von den Anwendern als angenehm empfunden wird.

Figur 2 zeigt schematisch die Behandlungsvorrichtung 1 gemäß Figur 1 in einer Draufsicht. Die Hochachse H ist hierbei als Achse dargestellt, welche senkrecht aus der Zeichenebene heraus oder in diese hinein verläuft. Die Querachse Q ist schematisch als rechtwinklig zur Außenseite bzw. Breitseite 70 des Verbindungssteges 14 dargestellt.

Es wird weiterhin deutlich, dass die freien Zahnenden 5 zumindest der Front- und gegebenenfalls der Eckzähne freibleiben.

Eine solche vorbeschriebene Behandlungsvorrichtung 1 ist selbstverständlich sowohl für einen Oberkiefer wie auch für einen Unterkiefer einsetzbar und verwendbar. Besonders vorteilhaft ist, dass eine der Befestigungseinrichtungen 10 (in Figur 2 die rechts dargestellte Befestigungseinrichtung 10) lediglich durch den Verbindungssteg 14 mit der anderen Befestigungseinrichtung 10 und der Anformung 20 verbunden ist, was eine leichte Beweglichkeit der ersten Befestigungseinrichtung 10 gegenüber der anderen Befestigungseinrichtung 10 in einer schematisch angedeuteten Doppelpfeilrichtung 80 bewirkt. Hierdurch kann in einfacher Art und Weise die Behandlungsvorrichtung an unterschiedliche Kiefergeometrien des Anwenders angepasst werden.

Dies eröffnet insbesondere die Möglichkeit, eine erfindungsgemäße Behandlungsvorrichtung 1 als Standardbehandlungsvorrichtung in großer Stückzahl herzustellen, da eine individuelle Anpassung einer solchen Behandlungsvorrichtung an konkrete Gegebenheiten, insbesondere geometrische Gegebenheiten im Mundraum eines Patienten durch die konstruktive Ausgestaltung der Behandlungsvorrichtung möglich ist. Somit können derartige Behandlungsvorrichtungen in lediglich einer oder zumindest in wenigen verschiedenen Grundgrößen hergestellt werden, um für eine Vielzahl unterschiedlicher Anwender anwendbar zu sein.

Des Weiteren kann es sinnvoll sein, gegebenenfalls die Wandungsabschnitte 12 und 13 zueinander biegbar oder verformbar auszugestalten, damit auch eine Anpassung an unterschiedliche Backenzahnbreiten und somit ein fester Sitz gewährleistet sein kann. Beispielsweise kann dies realisiert werden, wenn in den Befestigungseinrichtungen 10 Federbügel 100 oder z. B. Metallstreifen, die sich verformen lassen, vorhanden sind.

Im Übrigen haben sich als Materialien für die Behandlungsvorrichtung, zumindest für die Befestigungseinrichtungen 10, den Verbindungssteg 14 und die Anformung 20 medizinisch unbedenkliche Kunststoffe bewährt. Eine solche Behandlungsvorrichtung 1 kann beispielsweise im Wege des Tiefziehens (eher für die Kleinserie geeignet) oder im Wege des Spritzgusses hergestellt werden.

Insbesondere für eine Großserienherstellung eignet sich eine spritzgussgerechte konstruktive Ausgestaltung der Behandlungsvorrichtung 1. Insbesondere sind im Wege des Spritzgusses die Sensoreinrichtungen, Signalleitungen, die Steuerungs-/Regeleinheit, die Stimulationseinheit, die Energieversorgung und dergleichen in die Behandlungsvorrichtung 1 eingegossen oder anderweitig eingebettet. Als mögliche Werkstoffe zur Ausbildung der Behandlungsvorrichtung 1 bzw. zum Umgießen der anderen Bestandteile können beispielsweise medizinisch unbedenkliche Silikone, Kunststoffe und/oder Weichkunststoffe in Frage kommen.

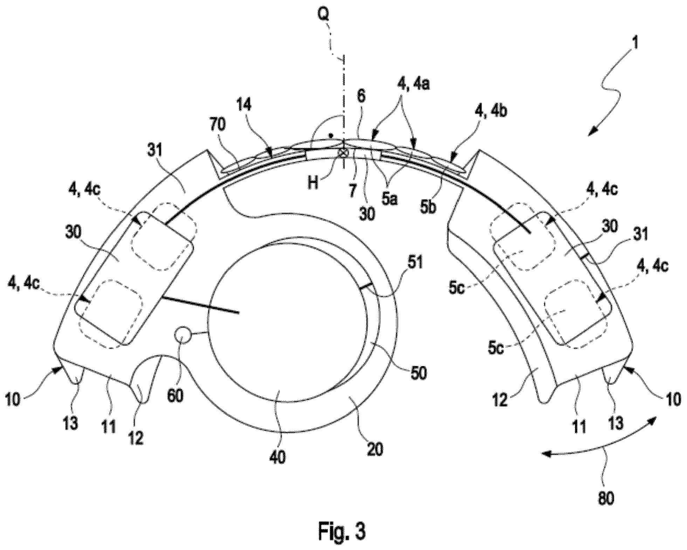

Figur 3 zeigt eine zweite Ausführungsform der erfindungsgemä-βen Behandlungsvorrichtung 1. Sie unterscheidet sich im Wesentlichen von der vorbeschriebenen Ausführungsform dadurch, dass der Verbindungssteg 14 im bestimmungsgemäßen Gebrauch der Behandlungsvorrichtung 1 an Innenseiten 7 z. B. von Front- und/oder Eckzähnen 4a, 4b unter Freilassung deren freier Enden 5a, 5b anliegt, insbesondere vorgespannt, z. B. gestaucht, anliegt. Bei einer solchen Ausgestaltung, die insbesondere für die Anwendung der Behandlungsvorrichtung 1 am Unterkiefer geeignet ist, kann zweckmäßigerweise eine Sensoreinrichtung 30 in den Verbindungssteg 14 integriert sein, um z. B. ein Zungenpressen zu detektieren, wenn eine Zunge von innen gegen die Zähne drückt.

Im Falle eines Einsatzes der Behandlungsvorrichtung 1 am Unterkiefer den Verbindungssteg 14 an Innenseiten der Zähne entlang laufen zu lassen, ist außerdem vorteilhaft, da beim Zubeißen ein physiologisch normaler Überbiss der oberen Zähen außerhalb der unteren Zähne stattfindet. Der Verbindungssteg 14 ist somit in einem für das Zubeißen nicht störenden Bereich. Gleiches gilt umgekehrt für die Anwendung der Behandlungsvorrichtung 1 am Oberkiefer. Dort ist es besonders zweckmäßig den Verbindungssteg 14 im bestimmungsgemäßen Gebrauch an freien Außenseiten 6 des Oberkiefers entlang laufen zu lassen, um zu verhindern, dass beim Zubeißen der Verbindungssteg 14 zwischen die Zähne des Oberkiefers und der entsprechenden Front- bzw. Eckzähne des Unterkiefers gelangt.

Mit der erfindungsgemäßen Behandlungsvorrichtung ist in einfacher Art und Weise eine Behandlungsvorrichtung geschaffen, die auf dem Effekt des sogenannten Biofeedbacks beruht und so gute Lerneffekte bzw. Behandlungserfolge erwarten lässt. Die erfindungsgemäße Behandlungsvorrichtung lässt sich in einfacher Art und Weise auf unterschiedlichste Indikationen (Zähneknirschen, Zungenpressen und/oder Schnarchen etc.) anpassen.

In besonders bevorzugter Ausführung ist die Behandlungsvorrichtung 1 als einstell- und konfigurierbares, insbesondere als regelbares Biofeedbacksystem auszugestalten, welches insbesondere eine Anpassung der Stimulation und der Auslöseempfindlichkeit und anderer Parameter, wie sie weiter unten in einem erfindungsgemäßen Betriebsverfahren beschrieben werden, anwenderindividuell oder an unterschiedliche Indikationen anwendungsspezifisch individuell anpassen lassen.

Obwohl die vorbeschriebene Behandlungsvorrichtung sich insbesondere für eine Großserienherstellung eignet und passend oder leicht anpassbar für die allermeisten Anwender ist, so kann die der Erfindung zu Grunde liegende Idee selbstverständlich auch auf individuell für den Anwender oder das Anwendungsgebiet hergestellte Behandlungsvorrichtungen übertragen werden und ohne weiteres dort eingesetzt werden.

In einer weiteren Ausführungsform der Erfindung können zusätzlich Speicherelemente vorgesehen sein, um beispielsweise die Häufigkeit und die Stärke des Zähneknirschens, Zungenpressens oder Schnarchens und die entsprechenden Reaktionen des Anwenders auf ausgeübte Stimulationen aufzuzeichnen. Selbstverständlich können hierfür zudem entsprechende Ausleseeinrichtungen, beispielsweise Bluetooth-Schnittstellen, Infrarot-Schnittstellen oder dergleichen vorgesehen sein.

Des Weiteren besteht die Möglichkeit, dass die Stimulation in Abhängigkeit von bestimmten Körperparametern oder Körpereigenschaften oder Körpermerkmalen des Anwenders geregelt und/oder gesteuert wird. In einem solchen Fall liegt es natürlich im Bereich der Erfindung entsprechende Sensoren, beispielsweise für die Körpertemperatur, für die Atemfrequenz, für den Blutdruck, für den Puls und dergleichen vorzusehen oder diese als externe Sensoren vorzusehen, welche mit der Steuerungs-/ und Regeleinheit entweder drahtgebunden oder über Funk in Verbindung stehen, sodass entsprechende derartige Regelgrößen von der Steuerungs-/Regeleinheit verarbeitet werden können.

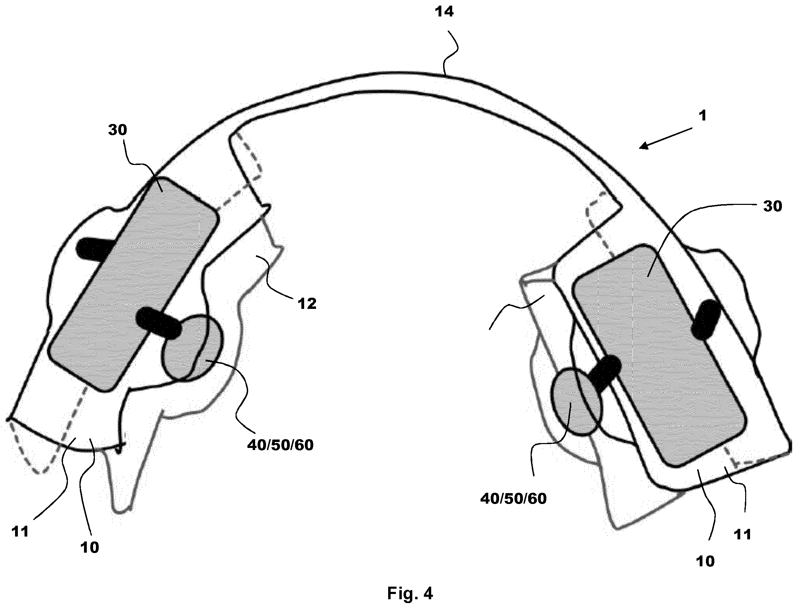

Eine dritte Ausführungsform der erfindungsgemäßen Behandlungsvorrichtung 1 ist in Figur 4 dargestellt. Dieser Ausführungsform der Behandlungsvorrichtung weist eine Vielzahl gleicher Bauteile/Funktionen auf, die auch in den vorbeschriebenen Ausführungsformen vorhanden sind. Insoweit werden gleiche Bauteile/Bestandteile mit gleichen Bezugszeichen versehen. Die vorangegangene Beschreibung gilt hier für diese Ausführungsform entsprechend.

Zur Freilassung des Gaumens des Benutzers der Behandlungsvorrichtung 1 schlägt die vorliegende Ausführungsform vor, die Steuerungs- und Regeleinheit 40, eine Stimulationseinrichtung 50 sowie die Energieversorgung 60 in dem inneren Wandungsabschnitt 12 der Befestigungseinrichtungen 10 einzubetten. Hierbei kann beispielsweise in jedem inneren Wandungsabschnitt 12 einer jeden Befestigungseinrichtung 10 jeweils eine Steuerungs-/Regeleinheit 40, eine Stimulationseinrichtung 50 sowie eine Energieversorgung 60 angeordnet sein. In einem solchen Fall ist jede Seite, d. h. linke und die rechte Seite der Behandlungsvorrichtung 1 autark betreibbar. Es kann eine Verbindung zwischen der linken Seite und der rechten Seite entfallen, so dass in dem Verbindungssteg 14 keine Signalleitung 31 eingebettet werden muss.

In der gezeigten Ausführungsform gemäß Figur 4 ist eine Anordnung aus Steuerungs-/Regeleinheit 40 der Stimulationseinrichtung 50 sowie der Energieversorgung 60 im inneren Wandungsabschnitt 12 eingebettet, d. h. zur Zunge des Benutzers bzw. zum Gaumen des Benutzers hin eingebettet.

Gleichwohl können die Bestandteile 40, 50, 60 natürlich auch in dem äußeren Wandungsabschnitt 13, d. h. wangenseitig im äu-βeren Abschnitt des Kiefers des Benutzers eingebettet sein.

Durch diese Maßnahme entsteht insbesondere ein höherer Freiraum für die Zunge bzw. im Bereich des Gaumens für den Benutzer, was oftmals als angenehm empfunden wird.

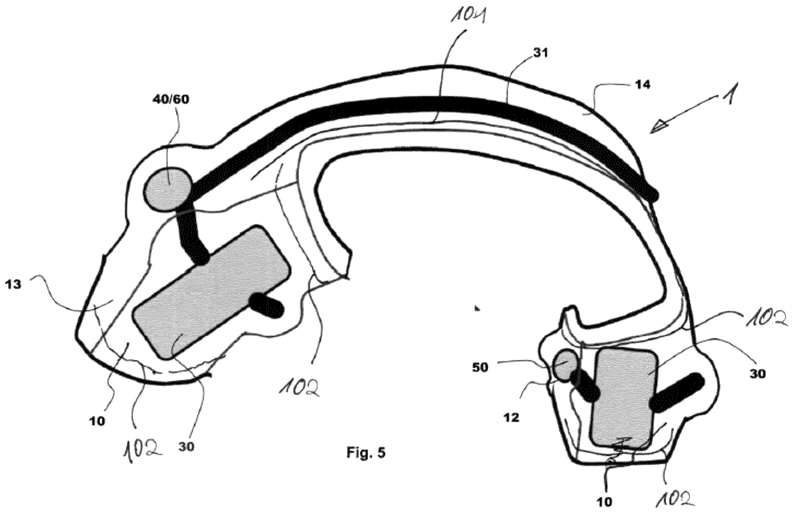

Eine weitere Ausführungsform der erfindungsgemäßen Behandlungsvorrichtung ist in Figur 5 schematisch dargestellt. Die Behandlungsvorrichtung 1 in der Ausführungsform gemäß Figur 5 zeigt eine Variante, bei der eine Signalleitung 31 im Verbindungssteg 14 eingebettet ist. Eine Stimulationseinrichtung 50 ist beispielsweise im Inneren Wandungsabschnitt 12 der ersten Befestigungseinrichtung 10 eingebettet. Eine Energieversorgung 60 und eine Steuerungs-/Regeleinheit 40 ist beispielsweise im äußeren Wandungsabschnitt 30 der zweiten Befestigungseinrichtung 10 angeordnet. Selbstverständlich sind neben den explizit gezeigten und beschriebenen Anordnungsvarianten auch alle anderen Varianten denkbar. So kann beispielsweise die Stimulationseinrichtung 50 durchaus auch im äußeren Wandungsabschnitt einer Befestigungseinrichtung 10 angeordnet sein, wohingegen die Steuerungs-/Regeleinheit 40 sowie die Energieversorgung 60 in einem Inneren Wandungsabschnitt 12 der anderen Befestigungseinrichtung 10 angeordnet bzw. eingebettet sein kann. Die vorliegenden Varianten können an die Bedürfnisse unterschiedlicher Patienten angepasst werden. Eine Anordnung der Bestandteile 40/50/60 in äußeren Wandungsabschnitten 13 bietet sich insbesondere beispielsweise bei Patienten mit sehr engen Kieferbögen an, die im Innenraum zwischen den Zahnreihen weniger Platz haben.

Des Weiteren ist auch eine Ausführungsform der Behandlungsvorrichtung 1 gemäß Figur 5 besonders für Patienten/Anwender geeignet, die es als unangenehm empfinden, am Gaumen einen Gegenstand anliegen zu haben.

Außerdem ist in der Figur 5 zeichnerisch noch eine weitere optionale Zusatzausstattung der Behandlungsvorrichtung dargestellt, die selbstverständlich auch auf alle vorbeschriebenen Ausführungsformen z. B. gemäß Figur 1, 2, 3 und 4 Anwendung finden kann. Diese Zusatzausstattung ist dort aber nicht zeichnerisch dargestellt.

Diese Zusatzausstattung betrifft alternativ oder kumulativ eine erste Versteifungseinrichtung 101 sowie weitere zweite Versteifungseinrichtungen 102. Diese als Versteifungseinrichtungen 101 und 102 bezeichneten Vorrichtungen können beispielsweise plastisch verformbare Drähte oder im Querschnitt anderweitig als rund ausgebildete Biegeleisten sein, die in das Trägermaterial der entsprechenden Bereiche der Behandlungsvorrichtung 1 eingebettet sind und somit möglichst nicht direkt mit dem Körper des Anwenders in Kontakt geraten. Die erste Versteifungseinrichtung 101 ist beispielsweise ein Draht oder eine anderweitige Versteifungseinrichtung, also z. B. ein Flachdraht, welcher entlang des Verlaufs des Verbindungssteges 14 benachbart zur Signalleitung 31 (sofern vorhanden) verläuft. Diese Versteifungseinrichtung 101 kann durch den Patienten leicht in Form gebogen werden, sodass die an sich relativ biegeschlaffe Ausbildung des Verbindungsstegs 14 eine gewisse Steifheit erlangt und somit die gesamte Behandlungsvorrichtung 1 an die individuellen Bedingungen (Radius des Kieferbogens des Anwenders) angepasst und entsprechend biegend eingestellt werden kann.

Im Prinzip gleichwirkend funktionieren die zweiten Versteifungseinrichtungen 102. Diese können ebenfalls plastisch verformbare Einrichtungen wie z.B. Runddrähte oder Flachdrähte sein, mittels denen die Weite/Breite der Befestigungseinrichtungen 10 durch plastisches Verbiegen der Befestigungseinrichtungen 10 eingestellt werden kann, sodass eine bessere Klemmung der Behandlungsvorrichtung 1 auf den Backenzähnen des Anwenders erzielbar ist. Die Versteifungseinrichtungen 102 können je Befestigungseinrichtung 10 auch mehrfach vorhanden sein, beispielsweise einmal von den Sensoreinrichtungen aus gesehen näher an den Frontzähnen und einmal näher an den hinteren Backenzähnen, sodass die Befestigungseinrichtungen 10 über ihre gesamte Längserstreckung besser in ihrer Form den individuellen Bedingungen eines Anwenders anpassbar sind.

Somit ist mit diesen Zusatzlösungen (Verstärkungseinrichtungen 101 und 102) eine verbesserte Anpassung der Behandlungsvorrichtung 1 an die individuellen Bedürfnisse des Anwenders möglich. Weiterhin ist die Steifigkeit der Behandlungsvorrichtung 1 als Ganzes verbessert, sodass die Passform weiter verbessert ist und sie besser auf den Zähnen hält. Ein geeigneter Flachdraht zum Einsetzen in den Verbindungssteg 14 hat beispielsweise eine Länge zwischen 60 mm und 85 mm, eine Höhe von 1,5 mm bis 3 mm und eine Dicke von 0,2 mm bis 0,5 mm.

Gegebenenfalls kann es auch zweckmäßig sein, als Versteifungseinrichtung 101 einen plastisch verbiegbaren Träger für die Verbindungsleitung 31 vorzusehen, sodass die Versteifungseinrichtung 101 und die Verbindungsleitung 31 vor dem Einbetten in das Trägermaterial der Behandlungsvorrichtung 1 (z. B. Silikon) als ein einziges plastisch durch Biegen verformbares Bauteil ausgebildet ist.

Ebenso kann es vorstellbar sein, die Versteifungseinrichtungen 102, welche zur Einbettung in die Befestigungsvorrichtungen 10 vorgesehen sind, bereits vor dem Einbetten in das Trägermaterial der Behandlungsvorrichtung 1 mit anderen Bauteilen, beispielsweise mit der Sensoreinrichtung, mechanisch zu verbinden, sodass der Einbettungsvorgang vereinfacht ist. Diese Maßnahmen stellen eine Vereinfachung der Herstellung der Behandlungsvorrichtung 1 dar.

Zur weiteren Verbesserung der Passform kann alternativ oder kumulativ zu obigen Maßnahmen auch vorgesehen sein, dass der Anwender bei einem Zahnarzt oder der Anwender selbst ein sogenanntes Unterfütterungssilikon unter die U-förmigen Befestigungseinrichtungen 10 einbringt und dieses "Unterfütterungssilikon" an die Zahnform, insbesondere die Zahnkronenform des Anwenders angepasst wird (z. B. durch "Einbeißen"). Somit kann ein Plateau gebildet werden, auf dem die Befestigungsvorrichtung 10 flächig und nicht nur punktuell aufliegt, sodass die individuelle Passform weiter verbessert ist. Gegebenenfalls macht es Sinn, dass nachträglich patientenindividuell angepasste "Unterfütterungssilikon" durch Kleben oder in anderer geeigneter Weise fest mit einer Unterseite der Befestigungsvorrichtung 10 innerhalb der U-förmigen Ausbildung zu verbinden.

### Bezugszeichenliste

- 1: Behandlungsvorrichtung
- 2: Kiefer
- 3: Gaumen
- 4: Zähne
- 4a: Frontzähne
- 4b: Eckzähne
- 4c: Backenzähne
- 5: freie Enden
- 5a: freie Enden der Frontzähne
- 5b: freie Enden der Eckzähne
- 5c: freie Enden der Backenzähne
- 6: Außenseiten
- 7: Innenseiten
- 7a: Zahnfleisch
- 10: Befestigungseinrichtungen
- 11: Wandungsabschnitt
- 12: innerer Wandungsabschnitt
- 13: äußerer Wandungsabschnitt
- 14: Verbindungssteg
- 20: Anformung
- 30: Sensoreinrichtung
- 31: Signalleitung
- 40: Steuerungs-/Regeleinheit
- 50: Stimulationseinrichtung
- 51: Verbindung
- 60: Energieversorgung
- 70: Außenseite/Breitseite
- 80: Doppelpfeilrichtung
- 100: Federbügel
- 101: erste Versteifungseinrichtung
- 102: zweite Versteifungseinrichtung
- H: Hochachse
- Q: Querachse

## Patentansprüche

1. Behandlungsvorrichtung zur intraoralen Anwendung zur Behandlung von Zähneknirschen und/oder Zungenpressen und/oder Schnarchen aufweisend:
- mindestens eine Sensoreinrichtung (30) ausgebildet und eingerichtet zur Detektion von Druckbelastungsereignissen und/oder Schallereignissen und/oder Schwingungsereignissen im Mundraum des Anwenders;
- mindestens eine Stimulationseinrichtung (50) zur Stimulation des Anwenders, wenn ein Druckbelastungsereignis und/oder ein Schallereignis und/oder ein Schwingungsereignis auftritt sowie
- eine Steuerungs- und/oder Regelungseinheit (40) zum gesteuerten und/oder geregelten Betreiben der Stimulationseinrichtung (50) in Abhängigkeit vom auftretenden Druckbelastungsereignis und/oder Schallereignis und/oder Schwingungsereignis sowie
- eine Energieversorgung
**dadurch gekennzeichnet, dass** sie aufweist zumindest zwei Befestigungseinrichtungen (10) zum Festlegen der Behandlungsvorrichtung (1) an mindestens jeweils einen Zahn (4) eines Oberkiefers und/oder Unterkiefers eines Anwenders, und dass die Befestigungseinrichtungen (10) mit mindestens einem biegsamen Verbindungssteg (14) miteinander verbunden sind, der dazu eingerichtet und ausgebildet ist, im bestimmungsgemäßen Gebrauch der Behandlungsvorrichtung (1) an Zahnaußenseiten (6) und/oder Zahninnenseiten (7) unter Freilassung von freien Zahnenden (5) der Zähne anzuliegen, wobei zumindest alle vorgenannten Bestandteile der Behandlungsvorrichtung (1) im bestimmungsgemäßen Gebrauch der Behandlungsvorrichtung (1) intraoral angeordnet sind und dass die mindestens eine Sensoreinrichtung (30) und/oder eine Signalleitung (31) im Verbindungssteg (14) integriert ist oder dass jeder Sensoreinrichtung (30) eine Steuerungs-/Regeleinheit (40) eine Stimulationseinrichtung (50) und eine Energieversorgung (60) zugeordnet ist, so dass jede Befestigungseinrichtung (10) eine autark funktionierende Anordnung aus Sensoreinrichtung (30), Steuerungs-/Regeleinheit (40), Stimulationseinrichtung (50) und Energieversorgung (60) besitzt.

2. Behandlungsvorrichtung nach Anspruch 1, **dadurch gekennzeichnet, dass** die Befestigungseinrichtungen (10) kappenartig oder im Querschnitt U-förmig nach Art einer U-Schiene ausgebildet und mindestens einen Zahn (4) übergreifend ausgebildet sind.

3. Behandlungsvorrichtung nach Anspruch 1 oder 2, **dadurch gekennzeichnet, dass** die Behandlungsvorrichtung (1) mindestens einen Wandungsabschnitt (11) besitzt, der im bestimmungsgemäßen Gebrauch zwischen Zähnen (4) des Oberkiefers und des Unterkiefers des Anwenders angeordnet ist und die mindestens eine Sensoreinrichtung (50) in diesem Wandungsabschnitt (11) integriert ist.

4. Behandlungsvorrichtung nach einem der vorangegangenen Ansprüche, **dadurch gekennzeichnet, dass** die Steuerungseinheit (40) und/oder die Regelungseinheit (40) und/oder die Stimulationseinrichtung (50) innenseitig in eine an zumindest einer der Befestigungseinrichtungen (10) angeformte Anformung (20) integriert oder eingekapselt, insbesondere eingegossen ist.

5. Behandlungsvorrichtung nach einem der vorangegangenen Ansprüche, **dadurch gekennzeichnet, dass** die Anformung (20) korrespondierend zu einem Gaumen (3) ausgeformt ist und/oder an einem Gaumen (3) zumindest teilweise anpassbar ausgebildet ist und/oder einem standardisierten Gaumen (3) entspricht, welches zu einer möglichst großen Anzahl von unterschiedlichen Anwendern passt.

6. Behandlungsvorrichtung nach einem der vorangegangenen Ansprüche, **dadurch gekennzeichnet, dass** der Verbindungssteg (14) bezüglich einer Krümmung entlang der Zahninnenseite (7) oder der Zahnaußenseite (6), d. h. um eine Hochachse (H) biegeweicher ausgestaltet ist, als um eine Querachse (Q) senkrecht auf eine Breitseite (70) des Verbindungssteges (14).

7. Behandlungsvorrichtung nach einem der vorangegangenen Ansprüche, **dadurch gekennzeichnet, dass** der Verbindungssteg (14) aus einem gummiartig dehn- und/oder stauchbaren Material ausgebildet ist und/oder einen Randbeschnitt aufweist, welcher ein Ziehharmonika-artiges Dehnen und/oder Stauchen erlaubt.

8. Behandlungsvorrichtung nach einem der vorangegangenen Ansprüche, **dadurch gekennzeichnet, dass** der Verbindungssteg (14) zugelastisch ausgebildet ist, wenn der Verbindungssteg (14) im bestimmungsgemäßen Gebrauch zur Anlage an der Zahnaußenseite (6) vorgesehen ist und/oder druckelastisch, stauchbar ausgebildet ist, wenn der Verbindungssteg (14) im bestimmungsgemäßen Gebrauch zur Anlage an der Zahninnenseite (7) ausgebildet ist.

9. Behandlungsvorrichtung nach einem der vorangegangenen Ansprüche, **dadurch gekennzeichnet, dass** die Behandlungsvorrichtung (1) keine gaumenseitige Anformung (20) aufweist und die Steuerungs-/Regeleinheit (40) und/oder die Stimulationseinrichtung (50) und/oder die Energieversorgung (60) in einen oder mehreren inneren Wandungsabschnitten (12) oder einen oder mehreren äußeren Wandungsabschnitten (13) eingebettet oder angeordnet sind.

10. Behandlungsvorrichtung nach einem der vorangegangenen Ansprüche, **dadurch gekennzeichnet, dass** der Verbindungssteg (14) keine Signalleitung (31) aufweist.

11. Behandlungsvorrichtung nach einem der vorangegangenen Ansprüche, **dadurch gekennzeichnet, dass** im Bereich des Verbindungssteges (14) und/oder im Bereich der Befestigungseinrichtungen (10) eine oder mehrere plastisch biegsam verformbare Versteifungseinrichtungen (101; 102) vorgesehen sind.

12. Behandlungsvorrichtung nach einem der vorangegangenen Ansprüche, **dadurch gekennzeichnet, dass** die Versteifungseinrichtungen (101; 102) ein plastisch verformbarer Draht oder Flachdraht sind.

## Claims

1. A therapeutic device for intraoral use for treatment of tooth grinding and/or tongue pressing and/or snoring, comprising:
- at least one sensor device (30) embodied and equipped to detect pressure load events and/or sound events and/or vibration events in a user's oral cavity;
- at least one stimulation device (50) for stimulating the user if a pressure load event and/or a sound event and/or a vibration event occurs, and
- a control and/or regulating unit (40) for controlled and/or regulated operation of the stimulation device (50) as a function of a pressure load event and/or sound event and/or vibration event that occurs as well as
- a power supply;
**characterized in that** the treatment device for intraoral use comprises at least two fastening devices (10) for fastening the therapeutic device (1) to at least one respective tooth (4) of the user's upper jaw and/or lower jaw, and that the fastening devices (10) are connected to each other with at least one flexible connecting piece (14), which is equipped and embodied, with proper use of the therapeutic device, to rest against outsides (6) of the user's teeth (6) and/or insides (7) of the user's teeth leaving uncovered free tooth ends (5) of the teeth, with at least all of the above-mentioned components of the therapeutic device (1) being positioned intraorally with proper use of the therapeutic device (1); and the at least one sensor device (30) and/or a signal line (31) is integrated into the at least one connecting piece (14), or each of the at least one sensor devices (30) is associated with a control/regulating unit (40), a stimulation device (50), and a power supply (60) so that each fastening device (10) has an independently functioning system composed of a sensor device (30), a control/regulating unit (40), a stimulation device (50), and a power supply (60).

2. The therapeutic device according to claim 1, **characterized in that** the fastening devices (10) are embodied as cap-like or U-shaped in cross-section like a U-rail and are embodied to embrace at least one tooth (4).

3. The therapeutic device according to claims 1 or 2, **characterized in that** the treatment device (1) has at least one wall section (11) which, with proper use, is positioned between teeth (4) of the user's upper jaw and lower jaw and the at least one sensor device (50) is integrated into this wall section (11).

4. The therapeutic device according to one of the preceding claims, **characterized in that** the control unit and/or the regulating unit (40) and/or the stimulation device (50) is integrated or encapsulated, in particular cast into, an inside of a molded protrusion (20) that is molded onto and extends inwardly from a single one of the at least one of the fastening devices (10).

5. The therapeutic device according to one of the preceding claims, **characterized in that** the molded protrusion (20) is molded to correspond to a palatine arch (3) and/or is embodied to be at least partially adaptable to a palatine arch (3) and/or is adapted to a standardized palatine arch (3) that fits a largest possible number of different users.

6. The therapeutic device according to one of the preceding claims, **characterized in that** the connecting piece (14) is more flexible with regard to a curvature along an inside (7) of the teeth or an outside (6) of the teeth, i.e. around a vertical axis (H), than around a transverse axis (Q) oriented perpendicular to a broad side (70) of the connecting piece (14).

7. The therapeutic device according to one of the preceding claims, **characterized in that** the connecting piece (14) is composed of a rubber-like stretchable and/or compressible material and/or has an edge section that permits an accordion-like stretching and/or compressing.

8. The therapeutic device according to one of the preceding claims, **characterized in that** the connecting piece (14) is tensile-elastic when the connecting piece (14) is placed on the outside (6) of the teeth with proper use and/or is pressure-elastic or compressible when the connecting piece (14) is placed on the inside (7) of the teeth with proper use.

9. The therapeutic device according to one of the preceding claims, **characterized in that** the therapeutic device (1)
has no palatine-side protrusion (20), and
the control unit and/or regulating unit (40) and/or the stimulation device (50) and/or the power supply (60) are embedded or arranged in at least one inner wall section (12) or at least one outer wall section (13).

10. The therapeutic device according to one of the preceding claims, **characterized in that** the connecting piece (14) does not have a signal line (31).

11. The therapeutic device according to one of the preceding claims, **characterized in that,** in a region of the connecting piece (14) and/or in a region of the fastening devices (10), one or more plastically-flexible, deformable reinforcing devices (101; 102) are provided.

12. The therapeutic device according to one of the preceding claims, **characterized in that** the reinforcing devices (101; 102) are a plastically deformable wire or flat wire.

## Revendications

1. Dispositif de traitement à usage intra-oral pour le traitement du grincement des dents et/ou du serrement de la langue et/ou du ronflement, comprenant :
- au moins un organe de détection (30) réalisé et conçu pour détecter des événements de charge en pression et/ou des événements sonores et/ou des événements vibratoires dans la cavité buccale de l'utilisateur ;
- au moins un organe de stimulation (50) pour stimuler l'utilisateur lorsqu'un événement de charge en pression et/ou un événement sonore et/ou un événement vibratoire se produit, et
- une unité de commande et/ou de régulation (40) pour le fonctionnement commandé et/ou régulé de l'organe de stimulation (50) en fonction de l'événement de charge en pression et/ou de l'événement sonore et/ou de l'événement vibratoire qui se produit, ainsi que
- une alimentation en énergie,
**caractérisé en ce que**
il comprend au moins deux organes de fixation (10) pour fixer le dispositif de traitement (1) à au moins une dent respective (4) de la mâchoire supérieure et/ou de la mâchoire inférieure d'un utilisateur,
et **en ce que** les organes de fixation (10) sont reliés entre eux par au moins une barrette de liaison (14) flexible qui, lors de l'utilisation conforme du dispositif de traitement (1), est conçue et réalisée pour s'appliquer contre les faces extérieures (6) et/ou les faces intérieures (7) des dents en laissant libres les extrémités libres (5) des dents, au moins tous les composants précités du dispositif de traitement (1) étant en particulier disposés intraoralement, lors de l'utilisation conforme du dispositif de traitement (1),
et **en ce que** ledit au moins un organe de détection (30) et/ou une ligne de signalisation (31) est intégré(e) dans la barrette de liaison (14),
ou **en ce que** chaque organe de détection (30) est associé à une unité de commande/régulation (40) et à un organe de stimulation (50) et à une alimentation en énergie (60), de sorte que chaque organe de fixation (10) possède un ensemble fonctionnant en autarcie constitué de l'organe de détection (30), de l'unité de commande/régulation (40), de l'organe de stimulation (50) et de l'alimentation en énergie (60).

2. Dispositif de traitement selon la revendication 1,
**caractérisé en ce que** les organes de fixation (10) sont réalisés en forme de capuchon ou en forme de U en section transversale à la manière d'un rail en U et sont réalisés de manière à coiffer au moins une dent (4).

3. Dispositif de traitement selon la revendication 1 ou 2,
**caractérisé en ce que** le dispositif de traitement (1) possède au moins une portion de paroi (11) qui, lors de l'utilisation conforme, est disposée entre les dents (4) de la mâchoire supérieure et de la mâchoire inférieure de l'utilisateur, et ledit au moins un organe de détection (50) est intégré dans ladite portion de paroi (11).

4. Dispositif de traitement selon l'une des revendications précédentes, **caractérisé en ce que** l'unité de commande (40) et/ou l'unité de régulation (40) et/ou l'organe de stimulation (50) est intégré(e) ou encapsulé(e), en particulier par coulée, du côté intérieur dans un surmoulage (20) formé sur au moins l'un des organes de fixation (10).

5. Dispositif de traitement selon l'une des revendications précédentes, **caractérisé en ce que** le surmoulage (20) est formé de manière à correspondre à un palais (3) et/ou est réalisé de manière à pouvoir être adapté au moins partiellement à un palais (3) et/ou correspond à un palais (3) standardisé qui convient au plus grand nombre possible d'utilisateurs différents.

6. Dispositif de traitement selon l'une des revendications précédentes, **caractérisé en ce que**, quant à une courbure le long de la face intérieure (7) de la dent ou le long de la face extérieure (6) de la dent, c'est-à-dire autour d'un axe vertical (H), la barrette de liaison (14) est conçue de manière plus souple qu'autour d'un axe transversal (Q) perpendiculaire à un côté large (70) de la barrette de liaison (14).

7. Dispositif de traitement selon l'une des revendications précédentes, **caractérisé en ce que** la barrette de liaison (14) est réalisée en un matériau extensible et/ou comprimable à la manière du caoutchouc et/ou présente une découpe de bord qui permet une extension et/ou une compression à la manière d'un accordéon.

8. Dispositif de traitement selon l'une des revendications précédentes, **caractérisé en ce que** la barrette de liaison (14) est réalisée de manière élastique à la traction lorsque la barrette de liaison (14) est prévue pour s'appliquer contre la face extérieure (6) de la dent, lors d'une utilisation conforme, et/ou est réalisée de manière élastique à la compression et comprimable lorsque la barrette de liaison (14) est conçue pour s'appliquer contre la face intérieure (7) de la dent, lors d'une utilisation conforme.

9. Dispositif de traitement selon l'une des revendications précédentes, **caractérisé en ce que** le dispositif de traitement (1) ne présente pas de surmoulage côté palais (20), et l'unité de commande/régulation (40) et/ou l'organe de stimulation (50) et/ou l'alimentation en énergie (60) sont encastrés ou disposés dans une ou plusieurs portions de paroi intérieures (12) ou dans une ou plusieurs portions de paroi extérieures (13).

10. Dispositif de traitement selon l'une des revendications précédentes, **caractérisé en ce que** la barrette de liaison (14) ne présente pas de ligne de signalisation (31).

11. Dispositif de traitement selon l'une des revendications précédentes, **caractérisé en ce qu'**il est prévu, dans la zone de la barrette de liaison (14) et/ou dans la zone des organes de fixation (10), un ou plusieurs organes de rigidification (101 ; 102) déformables plastiquement en flexion.

12. Dispositif de traitement selon l'une des revendications précédentes, **caractérisé en ce que** les organes de rigidification (101 ; 102) sont un fil métallique ou un fil métallique plat déformable plastiquement.
